# EUROPÄISCHE PATENTSCHRIFT

(11) **EP 2 681 263 B1**
(45) Veröffentlichungstag und Bekanntmachung des Hinweises auf die Patenterteilung: **12.09.2018**
(21) Anmeldenummer: 12705867.5
(22) Anmeldetag: 27.02.2012
(51) Int. Cl.: C08J 3/12, C08J 3/21, A61K 9/28, C08J 3/215, C08K 5/00

(54) **HERSTELLUNG VON PULVERFÖRMIGEN ÜBERZUGSMITTELN FÜR STABILE PROTEKTIVE ÜBERZÜGE FÜR PHARMAZEUTISCHE DOSIERUNGSFORMEN**
PROCESS FOR MAKING POWDER COATING COMPOSITIONS FOR STABLE PROTECTING COATING FOR PHARMACEUTICAL DOSAGE FORMS
PROCÉDÉ DE PRÉPARATION DE COMPOSITIONS DE REVÊTEMENTS EN POUDRE POUR DES REVÊTEMENTS PROTECTEURS STABLES DE FORMES DE DOSAGE PHARMACEUTIQUE

(30) Priorität: 28.02.2011 EP 11156224
(43) Veröffentlichungstag der Anmeldung: 08.01.2014
(73) Patentinhaber: BASF SE, 67056 Ludwigshafen am Rhein (DE)
(72) Erfinder: KOLTER, Karl, 67117 Limburgerhof (DE); ANGEL, Maximilian, 67105 Schifferstadt (DE); LINNER, Bernhard, 67240 Bobenheim-Roxheim (DE)
(74) Vertreter: BASF IP Association
(86) Internationale Anmeldenummer: PCT/EP2012/053231
(87) Internationale Veröffentlichungsnummer: WO 2012/116940

(56) Entgegenhaltungen:
- WO-A1-2011/012335
- WO-A2-2011/051155
- US-A1- 2005 079 216
- US-A1- 2005 271 778
- US-A1- 2005 281 871
- US-A1- 2011 033 532

## Beschreibung

Die vorliegende Erfindung betrifft die Herstellung von Überzugsmitteln in Pulverform für stabile protektive Überzüge für pharmazeutische Dosierungsformen, die zwecks Geschmacksmaskierung oder zum Schutz gegen Feuchtigkeit mit einem Filmüberzug auf Basis eines kationischen Polymers , welches mittels radikalischer Emulsionspolymerisation eines N,N-Diethylaminoethylmethacrylat enthaltenden Monomergemischs erhalten wird, versehen werden, durch Überführen einer wässrigen Dispersion mittels Sprühverfahren in ein freifliessendes Pulver mit guter Redispergierbarkeit.

Die DE-AS 2512238 lehrt zur Bereitstellung von Bindemitteln für Arzneimittelüberzüge mit geringem Restmonomerengehalt die Verwendung eines durch Sprühtrocknen einer Polymerdispersion erhaltenen Pulvers zur Herstellung von Überzugslösungen für diese Arzneiformen. Bezüglich der zur Sprühtrocknung eingesetzten Dispersionen wird auf die DE 1090381, DE 1219175 und DE 2135073 Bezug genommen.

Die DE 3049179 A1 ist eine Zusatzanmeldung zur DE 2512238 und betrifft die Verwendung eines durch Sprühtrocknen nach der Lehre des letztgenannten Dokuments gewonnenen Pulvers in Form einer wässrigen Suspension, die zusätzlich ein Weichmachungsmittel enthält, zur Herstellung von Überzügen durch Thermogelierung.

Die WO 00/05307 beschäftigt sich mit der Bereitstellung von Überzugs- und Bindemitteln für Arzneiformen, die (Meth)acrylat-Copolymere enthalten, die Monomerreste mit tertiären Aminogruppen aufweisen, wobei eine einfache trockene oder wässrige Weiterverarbeitung möglich sein soll. Dazu lehrt dieses Dokument ein Verfahren, bei dem man (a) ein Copolymer aus C₁-C₄-Estern der (Meth)acrylsäure und (Meth)acrylat-Monomeren, die tertiäre Ammoniumgruppen aufweisen, (b) einen Weichmacher und (c) einen Emulgator mit einem HLB-Wert von mindestens 14 miteinander vermengt und daraus das Überzugs- oder Bindemittel durch Schmelzen, Gießen, Ausstreichen oder Aufsprühen herstellt, wobei das Copolymer (a) in Pulverform mit einer mittleren Teilchengröße von 1 bis 40 µm eingebracht wird. Die dabei erzielte Verarbeitbarkeit wird der Bereitstellung des Copolymeren (a) in Pulverform mit extrem geringer Korngröße zugeschrieben.

Die WO 02/067906 betrifft Überzugs- und Bindemittel mit verbesserter Wasserdampfdurchlässigkeit gegenüber den in der WO 00/05307 beschriebenen. Dabei erfolgt die Herstellung der Überzugs- und Bindemittel mit einer Mischung, die (a) ein Copolymer aus C₁-C₄-Estern der (Meth)acrylsäure und weitere (Meth)acrylat-Monomere mit funktionellen tertiären Ammoniumgruppen in Pulverform mit einer mittleren Teilchengröße von 1 bis 40 µm, (b) einen Emulgator mit einem HLB-Wert von mindestens 14 und (c) eine C₁₂-C₁₈-Monocarbonsäure oder eine C₁₂-C₁₈-Hydroxylverbindung enthält.

Die WO 2004/019918 beschreibt Überzugs- und Bindemittel, die bezüglich ihrer Zusammensetzung den in der WO 00/05307 und WO 02/067906 beschriebenen entsprechen.

Gemäß der US 6,696,085 B2 soll ein Methacrylsäure-Copolymer Typ C als Zerfallsmittel eingesetzt werden. Das Methacrylsäure-Copolymer Typ C ist ein magensaft-resistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

In der EP88951 A2 ist ein Verfahren zum Überziehen von Arzneimitteln mittels eines in Wasser dispergierten Überzugsmittels auf Basis von Emulsionspolymerisaten beschrieben, wo bei die Überzugsmittel partiell in Salzform vorliegen können. Die Überzugsmittel können auch aus redispergierten Pulvern erhalten werden, wobei als grundsätzlich geeignete Methoden die verfahren der Sprühtrocknung und der Gefriertrocknung genannt werden . Allerdings wird in diesem Zusammenhang auch ausgeführt, dass die Gefriertrocknung auch an der unteren Grenze des Bereichs geeigneter Glastemperaturen noch anwandbar sei. Konkret beschreiben werden entweder durch Gefriertrocknung erhaltene Pulver oder ein sprühgetrocknetes Produkt aus 30 % Methacrylsäure und 70 % Methylmethacrylat, dass aufgrund seiner Zusammensetzung eine hohe Glasübergangstemperatur aufweist.

In der WO 97/42255 ist die Sprühtrocknung von in wässriger Lösung redispergierbaren Polymerpulvern, enthaltend freie säuren- oder basen-gruppentragende Copolymere, durch Sprühtrocknung beschrieben, wobei vor der Sprühtrocknung die pH-Werte der Dispersionen mit Hilfe eine Puffersystems eingestellt werden müssen.

In der EP 262326 A2 ist ein Verfahren zur Herstellung eines redispergierbaren Kunststoffpulvers beschrieben, bei dem eine wässrige Dispersion eines Copolymers aus (Meth)acrylsäure und (Meth)acrylsäureestern mit einer Mindestfilmbildetemperatur unter 60°C und einer dynamischen Einfriertemperatur unter 150°C so sprühgetrocknet wrid, dass die Eingangstemperatur des Trocknungsgases über der Mindestfilmbildetemperatur und unter der Einfriertemperatur liegt.

In der EP 901 787 A1 sind stabilisierte pharmazeutische Zubereitungen, umfassend gegen Zersetzung durch Licht oder freie Radikale empfindliche Wirkstoffe, beschrieben, die mit einem Beschichtungsmittel überzogen sind, das neben Metalloxiden, basischen Substanzen und Weichmachern freie Radikalfänger zur Stabilisierung der Wirkstoffe enthält. Als Basismaterialien für die Beschichtungsmittel sind alle grundsätzlich möglichen Materialien wie Zucker, Cellulose-derivate, Polyvinylpyrrolidone oder (Meth)acrylat-Polymere erwähnt. Es ist auch erwähnt, dass die Beschichtungsmittel als Retardpolymere Aminalkylmethycrylatpolymere enthalten können.

Aus der WO 2009/016258 ist die Herstellung der wässrigen Polymerdispersionen kationischer Polymere auf Basis von N,N-Diethylaminoethylmethacrylat wie sie erfindungsgemäß zum Einsatz kommen und deren Verwendung zum Überziehen von Arzneistoffen bekannt. Pulverförmige Überzugsmittel werden nur ganz allgemein erwähnt. Die Polymere zeigen aufgrund ihrer niedrigen Glasübergangstemperatur unter Umständen eine unerwünschte Neigung zur Agglomeration und stellen damit verarbeitungstechnisch hohe Anforderungen. Spezielle Methoden zur Stabilisierung beispielsweise gegen Verfärbung bei Lagerung oder zur Erzielung von Freisetzungsstabilität auch bei längerer Lagerung oder unter Stress werden nicht erwähnt.

In der US 2005/0281871 wird die Herstellung von pharmazeutischen Überzugsmitteln basierend auf einer Mischung aus zwei filmformenden Polymeren beschrieben, wobei als erstes Polymer ein tertiäre Aminogruppen tragendes Alkyl(meth)acrylat-Copolymer und als zweites Polymer ein anionische Gruppen tragendes Polymer eingesetzt werden. Die Überzugsmittel werden durch simultanes Versprühen von Lösungen oder Dispersionen, in denen die unterschiedlichen Polymere jeweils separat vorliegen, erhalten.

Der vorliegenden Erfindung liegt die Aufgabe zu Grunde, freifliessende pulverförmige Filmüberzugsmittel mit guter Redispergierbarkeit in Wasser zu finden, die für pharmazeutische Dosierungsformen, die auch bei längerer oder temperaturbelastender Lagerung keine Veränderung im Freisetzungsverhalten der Filmüberzüge und keine Verfärbung aufweisen, geeignet sind. Eine besondere Anforderung an die zu findenden redispergierten Überzugsmittel ist unter anderem die Erzeugung von stabilen kleinteiligen Dispersionen mit engen Teilchengrößenverteilungen und die Vermeidung von Koagulatbildung. Weiterhin soll auch eine Einarbeitung von stabilisierenden Substanzen wie Antioxidantien, die aufgrund der Schwerlöslichkeit in Wasser, die solche stabilisierenden Substanzen häufig aufweisen, gewährleistet werden, ohne dass die Stabilität der Polymerdispersion oder der Polymerpulver und deren anwendungs-technischen Eigenschaften negativ beeinflusst werden. Ein weiteres dabei zu lösendes Problem besteht darin, die meist schlecht in Wasser löslichen Antioxidantien nicht nur homogen in die wässrige Dispersion einzubringen, sondern auch ausreichend mit den dispergierten Polymerteilchen in Kontakt zu bringen mit dem Ziel, dass die in Wasser unlöslichen Antioxidantien in die Polymerteilchen wandern, da sie dort ihre Wirkung entfalten sollen. Somit musste ein Verfahren gefunden werden, das einen in Wasser schlecht löslichen Stoff über eine solche Wasserphase in eine andere Phase transportiert. In besonderer Weise wird das Problem dadurch verstärkt, dass kein Weichmacher verwendet werden kann, der als Schlepper fungieren könnte, da Weichmacher die Überführung einer solchen Zubereitung in ein redispergierbares Pulver aufgrund von einsetzender Verfilmung unmöglich macht.

Demgemäß wurde ein Verfahren zur Herstellung von pulverförmigen, Antioxidantien enthaltenden Überzugsmitteln aus wässrigen Polymerdispersionen, enthaltend als Komponente A ein durch radikalische Polymerisation erhaltenes Polymer aus
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,
gefunden, welches dadurch gekennzeichnet ist, dass die Antioxidantien in Form einer Lösung oder einer feinteiligen Dispersion in die wässrige Polymerdispersion eingearbeitet werden und die wässrige Dispersion durch Sprühverfahren in Pulverform überführt wird.

Die Antioxidantien werden in Form einer "feinteiligen Dispersion" eingebracht, wobei erfindungsgemäß "feinteilig" bedeutet, dass die mittlere Teilchengröße kleiner 20 µm beträgt.

Weiterhin wurde ein Verfahren gefunden, die mit Antioxidantien stabilisierte wässrige Polymerdispersion durch Sprühverfahren in freifliessende Pulver zu überführen, welches dadurch gekennzeichnet ist, dass die wässrige Polymerdispersion durch Sprühverfahren in Gegenwart eines Trocknungsgasesin freifliessende Pulver überführt wird, wobei die Eingangstemperatur des Trocknungsgases in die Sprühvorrichtung mindestens 20°C über der Glasübergangstemperatur und mindestens 20°C über der Mindestfilmbildetemperatur des Polymers liegt und die Ausgangstemperatur des Trocknungsgases aus der Sprühvorrichtung bei 40 bis 85°C gehalten wird.

Gemäß einer weiteren Ausführungsform liegt die Eingangstemperatur des Trockengases mindestens 20°C über der Glasübergangstemperatur und mindestens 20°C über der dynamischen Einfriertemperatur und mindestens 20°C über der Mindestfilmbildetemperatur des Polymers und wobei die Ausgangstemperatur des Trocknungsgases aus der Sprühvorrichtung bei 40 bis 85°C beträgt.

Vorzugsweise liegt die Eingangstemperatur des Trocknungsgases in die Sprühvorrichtung mindestens 40°C über der Glasübergangstemperatur und mindestens 40°C über der Mindestfilmbildetemperatur des Polymers.

Gemäß einer weiteren Ausführungsform liegt die Eingangstemperatur des Trockengases mindestens 40°C über der Glasübergangstemperatur und mindestens 40°C über der dynamischen Einfriertemperatur und mindestens 40°C über der Mindestfilmbildetemperatur des Polymers.

Für alle oben genannten Ausführungsformen beträgt die Ausgangstemperatur des Trocknungsgases vorzugsweise 45 bis 70°C

Gemäß einer weiteren bevorzugten Ausführungsform erfolgt die Überführung in freifliessende Pulver durch agglomerierende Sprühtrocknung.
Gemäß einer weiteren bevorzugten Ausführungsform werden die Polymere vor oder nach dem Sprühverfahren mit Säuren teilneutralisiert.

Eine weitere Ausführungsform betrifft die Durchführung eines Sprühverfahrens in Gegenwart von weiteren Polymeren und/oder weiteren Hilfsstoffen.

Die so erhaltenen Pulver können als pharmazeutische Überzugsmittel verwendet werden. Solche Überzugsmittel können durch Redispergierung in Wasser erhalten werden, wobei das durch ein Sprühverfahren erhaltene Pulver unter Einsatz von niedrig-scherenden Rührvorrichtungen bei Umdrehungen bis 1000 Upm redispergiert wird. Überraschenderweise können auch hoch-scherende Dispergiervorrichtungen bei Umdrehungen von > 5000 Upm verwendet werden. Dies kann erfolgen, ohne dass die bei der Redisperdierung gebildeten feinen Teilchen agglomerieren und die Zubereitung koaguliert.
Freifliessende Pulver im Sinne der vorliegenden Erfindung bedeutet, dass die Pulver bei der Bestimmung der Fließfähigkeit nach der DIN ISO 4324 unter Verwendung des Gerätes nach Pfrengle ohne Rührhilfe frei und vollständig aus dem Trichter ausfließen.
Polymerpulver erhalten gemäß einem der Ansprüche 1 bis26, bestehen aus einer Polymerdispersion enthaltend als Komponente A ein durch radikalische Polymerisation erhaltenes Polymer aus
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,
und Antioxidantien, wobei die mittlere Teilchengröße des in Wasser redispergierten Polymerpulvers maximal das 5-fache, bevorzugt maximal das 3-fache, besonders bevorzugt maximal das 2-fache, der zugrundeliegenden Primärdispersion beträgt.
Mittlere Teilchengrößen beziehen sich dabei auf das Intensitätsmittel ermittelt durch Lichtstreuung
Die für die Sprühverfahren verwendeten Überzugsmittel basieren auf wässrigen Polymerdispersionen, welche durch radikalische Emulsionspolymerisation eines Monomergemischs M), enthaltend
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,
in einem wässrigen Medium bei einem pH-Wert von wenigstens 8, erhalten werden.

Die Überzugsmittel in Form von wässrigen Polymerdispersionen enthalten vorzugsweise keine zusätzlichen organischen Lösungsmittel.

Die Überzugsmittel dienen zur Herstellung von pharmazeutischen Dosierungsformen, die im sauren Milieu des Magens schnell freisetzend sein sollen. D.h. die erfindungsgemäßen Überzüge sind magensaftlöslich. Schnell freisetzend bedeutet in diesem Zusammenhang, dass nach 60 min mindestens 80 % des Wirkstoffs freigesetzt sind. Erfindungsgemäß erhaltenen Überzüge sollen sich in Mundhöhle und Rachen im neutralen oder nahezu neutralen Milieu des Speichels nicht auflösen.
Die erfindungsgemäßen Überzugsmittel können zur Geschmacksmaskierung oder zum Schutz vor Feuchtigkeit verwendet werden. Die Wasserdampfpermeabilität der Überzüge ist sehr niedrig, wodurch feuchtigkeitsempfindliche Wirkstoffe geschützt werden.

Zur Herstellung der Polymerisate durch radikalische Emuslionspolymerisation wird hiermit ausdrücklich auf die Offenbarung der WO 2009/016258 Bezug genommen, in der die Herstellung und bevorzugte Ausführungsformen bezüglich Herstellung sowie Zusammensetzung ausführlich beschrieben sind.

In einer besonders bevorzugten Ausführungsform des erfindungsgemäßen Verfahrens wird eine Polymerdispersion eingesetzt, die aus einem Monomergemisch M), das aus
- 43 bis 47 Gew.- %, bezogen auf das Gesamtgewicht der zur Polymerisation eingesetzten Monomere, N,N-Diethylaminoethylmethacrylat a), und
- 53 bis 57 Gew.- %, bezogen auf das Gesamtgewicht der zur Polymerisation eingesetzten Monomere, wenigstens einer Verbindung b), insbesondere Methylmethacrylat,
besteht, erhalten wird.

Die in den Dispersionen enthaltenen Polymere weisen vorzugsweise ein mittels Gelpermeationschromatographie bestimmtes mittleres Molekulargewicht M_{w} im Bereich von 30000 bis 500000, besonders bevorzugt 60000 bis 140000, insbesondere 80000 bis 120000 g/mol, auf.

Die in den Dispersionen Pd) enthaltenen Polymere weisen vorzugsweise einen K-Wert (bestimmt nach Fikentscher an einer 1%igen Lösung in N-Methylpyrrolidon (NMP)) im Bereich von 40 bis 60 auf.

Die Glasübergangstemperatur T_{G} bestimmt mittels DSC "Differential Scanning Calorimetry" liegt vorzugsweise in einem Bereich von 40 bis 70°C, besonders bevorzugt 52 bis 62°C. Dabei werden die Proben zunächst auf 150°C aufgeheizt und dann von 150°C schnell abgekühlt. Die Messung der Glasübergangstemperatur erfolgt mit einer Heizrate von 20°K/min.
Die Mindestfilmbildetemperatur wird nach der in der DIN ISO 2115 beschriebenen Methode bestimmt und liegt im Bereich von 40 bis 70°C, vorzugsweise 50 bis 65°C. Die Messgenauigkeit der Methode liegt im Bereich von plus/minus 5°C.

Bei den in den Dispersionen enthaltenen Polymeren handelt es sich im Wesentlichen um statistische Copolymere.

Der mittlere Teilchendurchmesser der in der Polymerdispersion enthaltenen Polymerteilchen (bestimmt mittels analytischer Ultrazentrifuge) liegt vorzugsweise in einem Bereich von 70 bis 200 nm, besonders bevorzugt von 80 bis 150 nm, insbesondere von 90 bis 120 nm Die Teilchengrößenverteilung ist vorzugsweise im Wesentlichen unimodal.

Der LD-Wert der erfindungsgemäßen Dispersionen, bestimmt an einer 0,01%igen Dispersion in Wasser (2,5 cm Küvette, weißes Licht) beträgt vorzugsweise wenigstens 70 %, besonders bevorzugt wenigstens 80 %. Die Bestimmung der Lichtdurchlässigkeit wird z. B. in Dieter Distler, Wässrige Polymerdispersionen, Wiley-VCH (1999), S. 40, beschrieben.

Der Feststoffgehalt der erfindungsgemäß für die Sprühverfahren eingesetzten Dispersionen beträgt vorzugsweise 10 bis 50 Gew.-%, besonders bevorzugt 20 bis 40 Gew.-%. Im Falle einer vorherigen Reinigung der Dispersion mittels Ultrafiltration weisen die erfindungsgemäß eingesetzten Dispersionen vorzugsweise vor und nach der Ultrafiltration Feststoffgehalte auf, die in diesen Bereichen liegen. Selbstverständlich ist es ebenfalls möglich, eine verdünnte Polymerdispersion vor dem Sprühverfahren einer Aufkonzentrierung durch Ultrafiltration zu unterziehen.

Die zur Geschmacksmaskierung verwendeten redispergierbaren erfindungsgemäß hergestellten Polymerpulver weisen in Wasser bei einem Feststoffgehalt von 20 Gew.-% niedrige Viskositäten von vorzugsweise kleiner 300 mPas, besonders bevorzugt kleiner 200 mPas und insbesondere kleiner 100 mPas auf (Werte bestimmt mittels Brookfield-Viskosimeter bei 20 °C und 100 s⁻¹). Solche Viskositäten sind für viele Anwendungen von besonderer Bedeutung. Erfindungsgemäß erfolgt die Überführung der wässrigen Polymerdisperionen in die Pulverform mittels Sprühverfahren. Als Sprühverfahren eignen sich prinzipiell die Sprühtrocknung, die agglomerierende Sprühtrocknung, die Sprühgranulation (Sprühwirbelschichttrocknung) oder die Sprühagglomeration.

Die im Folgenden genannten Bedingungen für die Durchführung der Zerstäubung und Trocknung beziehen sich auf alle prinzipiell durchführbaren Ausführungsformen des Sprühverfahrens, sei es normale Sprühtrocknung, Sprühgranulation oder agglomerierende Sprühtrocknung.

Die Zerstäubung erfolgt vorzugsweise als hydrodynamische Zerstäubung durch Flüssigkeitsdruck oder Luftdruck über Düsen wie beispielsweise Einstoff- oder Mehrstoff-Düsen oder über Zerstäubungsscheiben.

Als Sprühvorrichtungen eignen sich herkömmliche Sprühtürme, in die die zu zerstäubende Polymerdispersion von oben eingeführt wird. Die erhaltenen Polymerpulver können am unteren Ende ausgetragen werden und in einem nachgeschalteten Zyklon vom Trocknungsgasstrom abgeschieden werden.

Als Trocknungsgase können Luft oder inerte Gase wie Stickstoff, Argon oder Helium verwendet werden. Die Trocknungsgase können im Gegenstrom oder im Gleichstrom zu den durch die Zerstäubung erzeugten Flüssigkeitströpfchen durch die Sprühvorrichtung geleitet werden. Vorzugsweise wird das Trocknungsgas im Gleichstrom eingesetzt. Die Eingangstemperatur des Trocknungsgases wird mindestens 20 °C, vorzugsweise mindestens 40 °C über der Glasübergangstemperatur und gemäß einer Ausführungsform auch mindestens 20°C bevorzugt mindestens 40 °C, über der dynamischen Einfriertemperatur und mindestens 20°C, bevorzugt mindestens 40 °C, über der Mindestfilmbildetemperatur des Polymers gehalten. Die Eingangstemperatur des Trocknungsgases in die Sprühvorrichtung wird besonders bevorzugt bei 100 bis 140 °C und die Ausgangstemperatur des Trocknungsgases aus der Sprühvorrichtung bei 45 bis 70 °C gehalten. Ganz besonders bevorzugt wird die Eingangstemperatur des Trocknungsgases in die Sprühvorrichtung bei 110 bis 130 °C und die Ausgangstemperatur des Trocknungsgases aus der Sprühvorrichtung bei 50 bis 60 °C gehalten. Die Ausgangstemperatur des Trocknungsgases liegt ganz besonders bevorzugt im gleichen Temperaturbereich plus/minus 5 °C wie die Mindestfilmbildetemperatur.

Die Verdampfung des Wassers in der Sprühvorrichtung kann sowohl bei Normaldruck als auch bei 0.06 bis 0.12 MPa erfolgen.

Bei der Durchführung der Sprühverfahren können den wässrigen Polymerdispersionen auch polymere Sprühhilfsmittel wie Polyvinylalkohole, Mischungen aus Polyvinylalkohol und einem aus Polyethylenglykol als Pfropfgrundlage und Polyvinylalkohol-Seitenketten bestehenden Pfropfcopolymer (kommerziell verfügbar als Kollicoat® Protect), Polyvinylpyrrolidone, alkylierte und/oder hydroxyalkylierte Cellulosen, Stärkederivate, Ligninsulfonate, Polyacrylsäuren oder Polyacrylamide zugegeben werden. Geeignete Mengen solcher Sprühhilfsmittel liegen im Bereich von 0,1 bis 30, vorzugsweise 1 bis 10 Gew.-%, bezogen auf den Feststoffgehalt.

Weiterhin können den wässrigen Polymerdisperionen auch Antiblockiermittel zugegeben werden. Geeignete Antiblockiermittel sind z.B. Aluminium-Silikate wie Bentonit, weiterhin Kieselgur, kolloidale Kieselsäure, gefällte Kieselsäure, Diaatomeenerde, Calciumcarbonat, Titandioxid, Zinkoxid, Magnesiumsilikate wie Talkum oder Tricalciumphosphat. Geeignete Mengen solcher Antiblockiermittel liegen im Bereich von 0,1 bis 15, vorzugsweise 0,5 bis 5 Gew.-%, bezogen auf den Feststoffgehalt.

Prinzipiell können den wässrigen Polymerdispersionen auch übliche Coatinghilfsstoffe hinzugefügt werden. Geeignete Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Antiklebemittel, Stabilisatoren, Antioxidantien, Porenbildner, Neutralisierungsmittel, Glanzmittel, Farbstoffe, Pigmente, Desinfektions- oder Konservierungsmittel, Verdickungsmittel oder Weichmacher. Geeignete Hilfsmittel sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Wie bereits erwähnt betrifft eine Ausführungsform der Erfindung die konventionelle Sprühtrocknung, bei der die zu trocknende wässrige Polymerdispersion zerstäubt und im Gasstrom des Trocknungsgases getrocknet und auf diese Weise in Pulverform überführt wrid.

Gemäß einer weiteren Ausführungsform kann die Überführung in Pulver durch eine Sprühgranulierung erfolgen. Dazu wird die zu trocknende wässrige Polymerdispersion ebenfalls zerstäubt und die erzeugten Tröpfchen gelangen dann in einem Wirbelbett in Kontakt mit vorgelegten Saatpartikeln, Durch dieses Inkontaktbringen der Saatpartikel mit den Tröpfchen der wässrigen Polymerdispersion wachsen die Saatteilchen zu größeren Granulatteilchen heran, wobei sich eine zwiebelschalenarteige Struktur um das als Saatgut verwendete Teilchen ausbildet.

Gemäß einer bevorzugten Ausführungsform der Erfindung erfolgt die Überführung in die Pulverform mit Hilfe der agglomerierenden Sprühtrocknung. Dabei wird die Polymerdispersion in einem Sprühturm wie oben beschrieben zerstäubt, wobei gleichzeitig Feinstaub, der aus der Trocknungszone abgeführt wird, in die Zerstäubungszone, in der die wässrige Polymerdispersion in Form von feinen Tröpfchen vorliegt, eingeblasen wird. Die Feinstaubpartikel werden dabei zu größeren Aggregaten mit einer brombeerförmigen Struktur verklebt. Zusätzlich kann auch noch ein Wirbelbett angeschlossen werden, in dem der Wassergehalt der gebildeten Teilchen weiter reduziert werden kann. Die resultierenden Aggregate können Teilchengrößen von 150 bis 1000 µm bevorzugt von 200 bis 500 µm aufweisen. Auch bei dieser Ausführungssform wird die Eingangstemperatur mindestens 20°C und bevorzugt mindestens 40°C über der Glasübergangstemperatur, und gemäß einer Ausführungsform auch mindestens 20°C, bevorzugt mindestens 40°C, über der dynamischen Einfriertemperatur und mindestens 20°C bevorzugt mindestens 40°C über der Mindestfilmbildetemperatur des Polymers und die Ausgangstemperatur des Trocknungsgases aus der Sprühvorrichtung bei 40 bis 85°C vorzugsweise bei 45 bis 70°C des Polymers gewählt. Bevorzugt wird die Eingangstemperatur des Trocknungsgases in die Sprühvorrichtung bei 100 bis 140°C und die Ausgangstemperatur des Trocknungsgases aus der Sprühvorrichtung bei 45 bis 70°C gehalten. Besonders bevorzugt wird die Eingangstemperatur des Trocknungsgases in die Sprühvorrichtung bei 110 bis 130°C und die Ausgangstemperatur des Trocknungsgases aus der Sprühvorrichtung bei 50 bis 60°C gehalten. Die durch Sprühagglomeration erhaltenen brombeerförmigen Strukturen sind nahezu staubfrei und zeigen ein besonders vorteilhaftes Verhalten bei der Redispergierung.

Bei allen vorstehend genannten Ausführungsformen können während des Sprühprozesses Sprühhilfsmittel wie z.B. Aluminium-Silikate wie Bentonit, Kieselgur, kolloidale Kieselsäure, gefällte Kieselsäure, Diaatomeenerde, Calciumcarbonat, Titandioxid, Zinkoxid, Magnesiumsilikate wie Talkum oder Tricalciumphosphat in Mengen von 0,1 bis 15, vorzugsweise 0,5 bis 5 Gew.-%, bezogen auf das Polymerpulver in den Sprühtum eingeblasen werden.

Der Restlösemittelgehalt beträgt üblicherweise nicht mehr als 5 Gew.-%, bezogen auf den Feststoffgehalt des Pulvers.

Insgesamt richten sich die Teilchengrößen des durch Sprühverfahren entstehenden Pulvers nach der jeweiligen Ausführungsform. Bei einer normalen Sprühtrocknung können Teilchengrößen von 10 bis 150 µm erzielt werden. Bei einer Sprühgranulation wie beispielsweise einer Sprühwirbelschichttrocknung können größere Teilchengrößen von 150 bis zu 1000 µm erreicht werden. Bei der agglomerierenden Sprühtrocknung können Teilchengrößen von 150 bis 1000 µm erzielt werden.

Gemäß einer weiteren Ausführungsform werden dem Polymer Säuren zugesetzt. Vorzugsweise werden solche Mengen an Säure zugesetzt, dass die basischen Gruppen teilweise in Form der Säuresalze vorliegen. Bevorzugt werden 1 bis 20 mol-%, besonders bevorzugt 2 bis 15 mol-% der basischen Gruppen neutralisiert. Dies kann vor oder nach der Sprühtrocknung erfolgen. So kann beispielsweise die Säure vor der Sprühtrocknung zu der wässrigen Polymerdispersion gegeben werden. Gemäß einer anderen Ausführungsform kann die Säure auch vor oder während der Redispergierung zugesetzt werden. Erfolgt die Einarbeitung der Säure vor der Sprühtrocknung, so kann diese mit üblichen Verfahren in die wässrige Dispersion eingerührt werden. Bei Zugabe nach der Sprühtrocknung erfolgt die Einarbeitung der Säure in das Polymerpulver so, dass zunächst das Polymerpulver in Wasser mittels eines einfachen Rührers grob vordispergiert wird, anschließend die Säure zugesetzt wird und durch Weiterrühren die vollständige Redispergierung erreicht wird. Die Redispergierung ist sehr schnell und so liegen schon nach 10 min feinteilige Dispergate vor. In einer modifizierten orgehensweise kann auch zunächst die Säure in Wasser vorgelegt werden und hierzu unter Rühren das Polymerpulver gegeben werden. Ferner ist es möglich, zunächst Polymerpulver und Säure zu mischen und diese Pulvermischung in Wasser einzutragen.
Als Säuren eignen sich anorganische Säuren oder saure Salze wie Kohlensäure (Einpressen von Kohlendioxid), Ammoniumhydrogencarbonat, Natriumhydrogencarbonat, Salzsäure, Schwefelsäure oder Phosphorsäure oder Phosphorsäuresalze wie Natriumdihydrogenphosphat. Weiterhin eignen sich organische Säuren wie Weinsäure, Citronensäure, Milchsäure, Glykolsäure, Äpfelsäure, Malonsäure, Maleinsäure, Bernsteinsäure, Fumarsäure, Asparaginsäure, Glutaminsäure, Gluconsäure oder weitere physiologisch verträgliche Säuren. Selbstverständlich kommen auch polymere Säuren auf natürlicher und/oder synthetischer Basis in Frage. Die genannten Säuren eigenen sich für alle beschriebenen Ausführungsformen.

Gemäß einer weiteren Ausführungsform der Erfindung werden Säuren, die unter den Bedingungen des Sprühverfahrens zersetzt werden oder verdampfen, eingesetzt. Nach dieser Ausführungsform liegen die Polymere vor und während des Sprühverfahrens in neutralisierter oder teilneutralisierter Form vor, während im entstehenden Pulver wieder die freie basische Form vorliegt.

Welche Gewichtsmengen an Säuren im Einzelnen verwendet werden, richtet sich nach dem jeweiligen Molekulargewicht und dem oben beschriebenen angestrebten Neutralisationsgrad.

Bevorzugt wird die Behandlung mit Säuren so durchgeführt, dass der pH-Wert der wässrigen Dispersion, des Pulvers oder des in Wasser redispergierten Pulvers im Bereich von 5 bis 9 liegt.

Besonders bevorzugt erfolg die Zugabe der Säure oder des sauren Salzes so, dass der pH-Wert der wässrigen Dispersion, des Pulvers oder des in Wasser redispergierten Pulvers im Bereich von 6 bis 8 liegt.

Die Herstellung von Überzugsmitteln kann z. B. durch inniges Vermischen einer durch Redispergieren des erfindungsgemäß erhaltenen Polymerpulvers zu einer wässrigen Polymerdispersion, der vorzugsweise mindestens ein weiterer Hilfsstoff zugesetzt wird, erfolgen.

Zur Stabilisierung werden die Polymerdispersionen vor der Überführung in die Pulverform wie erwähnt mit in Wasser schwerlöslichen Antioxidantien behandelt. Der Begriff "Antioxidantien" ist dem Fachmann an sich bekannt (siehe z.B. Römpp - Lexikon der Chemie, 9. Auflage, 1989, Georg-Thieme-Verlag, Stuttgart) und bezeichnet Substanzen, die unerwünschte durch Sauerstoff oder andere oxidative Prozesse bedingte Veränderungen hemmen oder verhindern sollen. Als Antioxidantien zur Stabilisierung der Überzugsmittel eignen sich erfindungsgemäß in Wasser schwerlösliche Antioxidantien, also Antioxidantien deren Löslichkeit in Wasser bei 20 °C nicht mehr als 1 g/l beträgt.
Erfindungsggemäß eignen sich als Antioxidantien vor allem die lipophilen Substanzen Tocopherol, Tocopherolacetat, Ascorbylpalmitat, Ascorbylstearat, t-Butylhydrochinon, t-Butylhydroxyanisol, t-Butylhydroxytoluol, Octylgallat oder Dodecylgallat oder deren Kombinationen.

Einarbeiten der Antioxidantien in Form einer Lösung in einem organischen Lösungsmittel.

Gemäß einer Ausführungsform der Erfindung werden die Antioxidantien in einem organischen Lösungsmittel gelöst eingesetzt. Als organische Lösungsmittel eignen sich solche Lösungsmittel, die einerseits ausreichend mit Wasser mischbar sind, so dass eine Konzentration von mindestens 10 Gew.-% in Wasser erreicht werden kann, , andererseits aber auch in der Lage sind, die in Wasser schwerlöslichen Antioxidantien lösen. Als Lösungsmittel in Betracht kommen Alkohole wie z.B. Ethanol oder Isopropanol, Ketone wie z.B. Aceton, Methylethylketon und Ester wie z.B. Methylacetat. Üblicherweise weisen diese Lösungsmittel Siedepunkte unter 100°C auf.
Die Antioxidantien können auf an sich übliche Weise in organische Lösung gebracht werden. Die Konzentration wird so gewählt, dass 10 bis 1000g Antioxidans pro Liter Lösungsmittel eingesetzt werden. Insgesamt wird die Menge an organischem Lösungsmittel so gewählt, dass 1 bis 20 Gew.-% Lösungsmittel bezogen auf das Gewicht der wässrigen Dispersion eingesetzt werden.

Gemäß einer weiteren Ausführungsform können die Antioxidantien in Form einer wässrigen micellaren Lösung in die wässrige Dispersion eingearbeitet werden. Dazu werden die Substanzen in Gegenwart von solubilisierend wirkenden Substanzen ("Solubilisatoren") in Lösung gebracht (zum Begriff der "Solubilisierung" siehe Römpp-Chemielexikon, 9. Auflage). Als Solubilisatoren eigenen sich Tenside wie z.B. Natrium-Docusat oder Natriumdodecylsulfat, ethoxylierte Fette, ethoxylierte Fettsäuren, ethoxylierte Fettalkohole oder polymere Solubilisatoren.

Als polymere Solubilisatoren eignen sich vor allem amphiphile Copolymere. Unter amphiphilen Copolymeren werden erfindungsgemäß Copolymere verstanden, die aus hydrophilen und hydrophoben Segmenten aufgebaut sind. Die Segmente können auch eine LCST (Lower Critical Solution Temperature) aufweisen. Die Segmente stellen ihrerseits Polymerketten dar, die aufgrund ihrer Zusammensetzung bzw. der zur Herstellung der Segmente verwendeten Monomeren entweder hydrophil oder hydrophob sind. Die amphiphilen Copolymere können Blockpolymere oder Pfropfpolymere sein. Die Struktur der Copolymer kann neben linearen Blockpolymeren auch kammförmig oder sternförmig sein. Im Falle der Pfropfpolymere können entweder hydrophobe Seitenketten und eine hydrophile Pfropfgrundlage vorliegen oder hydrophile Seitenketten und eine hydrophobe Pfropfgrundlage. Die Seitenketten können sowohl angepfropft als auch aufgepfropft werden. Geeignete amphiphile Copolymere sind beispielsweise in der WO 2007/017452, der WO 2007/051743, der WO 2007/ 065845 und der WO 2007/065846 offenbart, auf deren Beschreibung hinsichlich geeigneter amphiphiler Copolymere und deren Herstellung hiermit Bezug genommen wird.. Weitere amphiphile Copolymere sind beispielsweise Poloxamere.

Als hydrophile Segmente eigenen sich N-Vinyllactam-Homo- oder Copolymerketten, insbesondere N-Vinylpyrrolidon-haltige Polymere ebenso wie Polyvinylalkohol-Ketten oder Polyether.

Als hydrophobe Segmente eignen sich beispielsweie Homo-oder Copolymere des N-Vinylacetats. Als Comonomer eignet sich beispielsweise N-Vinylcaprolactam.

Bevorzugt wird als polymerer Solubilisator ein kommerziell unter dem Namen Soluplus®, Firma BASF SE, erhältliches Pfropfcopolymer mit PEG 6000 als Pfropfgrundlage und einer Copolymerseitenkette hergestellt aus Vinylacetat und N-Vinylcaprolactam.

Weiterhin eignen sich zur Herstellung der micellaren Lösung alle Tenside, die einen HLB von über 12 aufweisen. Solche Tenside sind in "Fiedler, Encyclopedia of Excipients", Editio Cantor Verlag. Sixth edition, 2007, Seite 112 - 119, beschrieben.

Die wässrigen Antioxidans - Solubilisate enthalten 0,5 bis 30 Gew.-%, vorzugsweise 1 bis 20 Gew.-% Antioxidans und 1 bis 50 Gew.-%, vorzugsweise 1 bis 30 Gew.-% Solubilisator. Insgesamt wird die Menge so gewählt, dass 1 bis 40 Gew.-% wässriges Antioxidans - Solubilisat bezogen auf das Gewicht der wässrigen Dispersion eingesetzt werden

Gemäß einer weiteren Ausführungsform der Erfindung werden die in Wasser schwerlöslichen Antioxidantien in Form von feinteiligen wässrigen Dispersionen in die wässrige Dispersion des polymeren Überzugsmittels eingebracht. Als Dispersionen sind in diesem Zusammenhang zweiphasige Systeme bezeichnet, die entweder fest/flüssig (Suspensionen) oder flüssig/flüssig (Emulsionen) sein können. Die mittlere Teilchengröße (d4,3) der Antioxidantien sollte hierbei kleiner 20 µm, vorzugsweise kleiner 10 µm besonders bevorzugt kleiner 3 µm betragen.

So können die Antioxidantien in Emulgatoren gelöst und dann in Wasser dispergiert werden. Die Antioxidantien können aber auch direkt in Wasser gegeben werden und unter Zuhilfenahme von Emulgatoren mit hochscherenden Dispergierwerkzeugen dispergiert werden. Besonders bevorzugt ist hierbei die Erwärmung der Zubereitung auf eine Temperatur oberhalb des Schmelzpunktes des Antioxidans, wodurch eine Emulsion entsteht. Diese heiße Emulsion kann direkt unter Rühren zur Polymerdispersion hinzugefügt werden. Alternativ kann sie auch vorher abgekühlt werden, wodurch eine feinteilige Suspension entsteht. Besonders bevorzugt ist, die heiße Emulsion zu einer Polymerdispersion zu geben, die ebenfalls eine Temperatur oberhalb des Schmelzpunktes des Antioxidans aufweist.
Als Emulgatoren eignen sich prinzipiell alle Klassen von grenzflächenaktiven Substanzen mit einem HLB-Wert von > 10 (zum **H**ydrophilic-**L**ipophilic-**B**alance-Wert siehe Fiedler, Encyclopedia of Excipients, Editio Cantor Verlag Sixth edition, 2007, Seite 112 - 119). Als Emulgatoren eignen sich grundsätzlich alle ethoxilierten Fettsäuren, ethoxilierte Fettalkohole, ethoxilierte Fettsäureether oder ethoxilierte Fettsäureester mit entsprechenden HLB-Werten. Geeignet sind beispielsweise entsprechende ethoxilierte Sorbitan-, Stearyl, Oleyl-, Lauryl- oder Palmityl-Derivate, beispielsweise Solutol® HS (Macrogol 15 hydroxystearat) oder ethoxiliertes hydriertes Ricinusöl wie beispielsweise Cremophor® RH40 (ethoxiliert mit 40 Ethylenoxid-Einheiten) oder die entsprechenden Eumulgin®-Typen.
Weiterhin eignen sich als Emulgatoren Poloxamere (Polyethylenoxid-Polypropylenoxid-Blockcopolymere)

Die wässrigen Antioxidans - Emulgatordispersionen enthalten 1 bis 50 Gew.-%, vorzugsweise 2 bis 30 Gew.-% Antioxidans und 0,1 bis 30 Gew.-%, vorzugsweise 0,5 bis 10 Gew.-% Emulgator.
Insgesamt wird die Menge so gewählt, dass 1 bis 40 Gew.-% wässrige Antioxidans - Emulgatordispersion bezogen auf das Gewicht der wässrigen Polymerdispersion eingesetzt werden.

Gemäß einer bevorzugten Ausführungsform der Erfindung werden die Antioxidantien in Form einer sogenannten "festen Lösung" eingesetzt. Der Begriff "feste Lösung" ist dem Fachmann bekannt und bezeichnet eine molekulardisperse Verteilung eines Feststoffs in einem anderen Feststoff. Im vorliegenden Falle können die Antioxidantien als feste Lösungen in einen geeigneten festen Solubilisator oder in ein polymeres Schutzkolloid eingearbeitet werden. Die resultierende feste Lösung kann dann direkt in fester Form in die wässrige Überzugsmitteldispersion eingearbeitet werden oder vorher in eine micellare wässrige Lösung oder in eine kolloidale Lösung überführt und dann in die wässrige Überzugsmitteldispersion eingearbeitet werden. Die festen Lösungen können beispielsweise hergestellt werden, indem die Antioxidantien gemeinsam mit dem Solubilisator oder dem Schutzkolloid gemeinsam in einem geeigneten Lösungsmittel gelöst und anschliessend das Lösungsmittel verdampft werden.

Gemäß einer besonders bevorzugten Ausführungsform wird die feste Lösung durch Schmelzextrusion hergestellt, wobei Antioxidantien und Solubilisatoren oder polymere Schutzkolloide gemeinsam aufgeschmolzen und anschliessend extrudiert, geformt und verfestigt werden. Die nach der Extrusion erhaltenen granulären festen Schmelzextrudate lassen sich besonders vorteilhaft in die wässrige Dispersion des polymeren Überzugsmittels einarbeiten. Als Matrixpolymere und Schutzkolloide für feste Lösungen eignen sich hierbei die bereits erwähnten amphiphilen Copolymere, insbesondere Soluplus®, oder Poloxamere wie Lutrol® F86 aber auch nicht amphiphile Polymere wie z.B. Polyvinylpyrrolidone, Vinylpyrrolidon - Vinylacetat-Copolymere, Polyethylenglykole. Polyvinylalkohole, Polyvinylalkohol-Polyethylenglykol-Pfropfcopolymere oder hydroxyalkylierte Cellulosen.

Die Herstellung von Überzugsmitteln kann z. B. durch inniges Vermischen einer durch Redispergieren des erfindungsgemäß erhaltenen Polymerpulvers zu einer wässrigen Polymerdispersion, der vorzugsweise mindestens ein weiterer Hilfsstoff zugesetzt wird, erfolgen.

Gemäß einer bevorzugten Ausführungsform wird während oder nach dem Sprühverfahren dem entstehenden Polymerpulver Siliciumdioxid zugesetzt.

Geeignete zusätzliche Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Antiklebemittel, Stabilisatoren, Antioxidantien, Porenbildner, Neutralisierungsmittel, Glanzmittel, Farbstoffe, Pigmente, Desinfektions- oder Konservierungsmittel, Verdickungsmittel, Weichmacher etc. Solche Stoffe sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Zur Herstellung des Überzugsmittels wird das erfindungsgemäß hergestellte N,N-Diethylaminoethylmethacrylat-basierende Polymerpulver vor der Redisergierung in Wasser gemahlen. Die Mahlung kann auch in Gegenwart der genannten zusätzlichen Hilfsstoffe erfolgen.

Übliche Mengen der Hilfsstoffe liegen in einem Bereich von jeweils 0 bis 70 Gew.-%, bevorzugt 0 bis 60 Gew.-%, insbesondere 1 bis 50 Gew.-%, bezogen auf das Gesamtgewicht des Feststoffes des Überzugsmittels.

Das aus den erfindungsgemäß erhaltenen Pulvern erhaltene Überzugsmittel kann aber auch in Pulverform auf die pharmazeutischen Dosierungsformen aufgetragen werden.
Der Auftrag kann auch in wässriger Form durch Granulieren, Gießen, Ausstreichen oder mittels Sprühauftrag erfolgen.

Bevorzugt ist die Anwendung als durch Redispergierung erhaltene wässrige Polymerdispersion. Grundsätzlich eignet sich jede Dispergiervorrichtung für die Redispergierung. Dabei erfolgt die Redispergierung vorzugsweise unter Anwendung niedriger Scherkräfte, vorzugsweise mittels eines Blatt, Propeller, Ankerrührers oder eines vergleichbaren Rührwerkzeuges. Die erfindungsgemäßen Polymerpulver redispergieren hierbei spontan und schnell. Die Redispergierung der Polymerpulver in Wasser ist üblicherweise in 10 min abgeschlossen.

Diesen redispergierten Zubereitungen können für den Coatingauftrag erforderliche weitere Komponenten zugesetzt werden. Solche Komponenten sind insbesondere Weichmacher wie z.B. Triethylcitrat, Tributylcitrat, Diethylsebacat, Dibutylsebacat, Acetyltriethylcitrat.

Überraschenderweise überstehen die feinteiligen Dispersionen auch sehr hohe Scherkräfte wie beispielsweise in einer Rotor-Stator-Vorrichtung, die auch Ultra-turrax genannt wird oder einer Kolloidmühle. Die Eintragung hoher Scherkräfte wird in einer Rotor-Stator-Vorrichtung über die Umdrehungszahl der Vorrichtung geregelt. Vorzugsweise erfolgt die Redispergierung mit Hilfe einer Dispergiervorrichtung bei < 5000 Upm. Dieses Verfahren ist insbesondere von Vorteil wenn zusätzlich in die Dispersion weitere grobteilige Zusatzstoffe oder agglomerierte Zusatzstoffe eingearbeitet werden müssen, die einer besonderen Zerkleinerung bedürfen. Es entfällt somit die separate Zerkleinerung dieser Zusatzstoffe in Wasser und spätere Zugabe zum redispergierten Polymerpulver.

Aus den erfindungsgemäß erhaltenen redispergierbaren Polymerpulvern werden durch Vermischen mit weiteren üblichen vorstehend beschriebenen Coatingbestandteilen bzw. Zusatzstoffen sogenannte ready-to use Zubereitungen hergestellt, die alle erforderlichen Bestandteile eine Coatings enthalten. Diese liegen in Pulver- oder Granulatform vor. Der Anwender braucht diese zur Herstellung einer sprühfertigen Suspension nur noch in Wasser einzurühren. Die Herstellung dieser ready-to-use Zubereitungen erfolgt durch trockenes Mischen, Vermahlen, Kompaktieren oder Granulieren der Bestandteile mit einer Granulierflüssigkeit gefolgt von einem Abtrocknungsschritt. Insbesondere können auf diese Weise Säuren oder saure Salze, die die Redispergierung unterstützen, eingearbeitet werden.

Wenn nicht anders angegeben, handelt es sich bei allen Angaben im Rahmen der vorliegenden Erfindung zur mittleren Teilchengröße von Pulvern im Mikrometerbereich um das mittels Lichtbeugung bestimmte Volumenmittel der Teilchendurchmesser (d4,3-Wert).

Die Überzugsmittel können zusätzlich wenigstens eine weitere Polymerkomponente enthalten. Dabei können Mischungen aus wenigstens zwei Dispersionen, wenigstens einer Dispersion und wenigstens einer Lösung, wenigstens einer Dispersion und wenigstens einem Pulver, wenigstens zwei Pulvern, etc. zum Einsatz kommen.

Die Überzugsmittel eignen sich für Dosierungsformen grundsätzlich beliebiger pharmazeutischer Wirkstoffe, die vorzugsweise in isolierter oder geschützter Form verabreicht werden können, wie Antidepressiva, Betarezeptorenblocker, Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen-Darmtherapeutika, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika, Nutraceuticals, Vitamine, Carotinoide und Aminosäuren.

Beispiele geeigneter Wirkstoffe sind: Acarbose, Nichtsteroidale Antirheumatika, Herzglykoside, Acetylsalicylsäure, Virustatika, Aclarubicin, Aciclovir, Cisplatin, Actinomycin, α-und β-Sympatomimetika, Allopurinol, Alosetron, Alprostadil, Prostaglandine, Amantädin, Ambroxol, Amlodipin, Methotrexat, 5-Aminosalicylsäure, Amitriptylin, Amoxicillin, Anastrozol, Atenolol, Atorvastatin, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Celetoxib, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und. Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridamol, Domperidon und Domperidonderivate, Donepzil, Dopamin, Doxazosin, Doxorubicin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Esomeprazol, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Galantamin, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerofderivate, Hypothalamushormone, Goserelin, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolclerivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat, Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate ; Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Orlistat, Oseltamivir, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Rosiglitazon, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralin, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Tegaserod, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Antiöstrogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valdecoxib, Valproinsäure, Vancomycin, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zanamivir, Zidovudin, Zolmitriptan, Zolpidem, Zopiclon, Zotepin und dergleichen.

Die Wirkstoffe können gewünschtenfalls auch in Form ihrer pharmazeutisch akzeptablen Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemäßen Zusammensetzungen auch zwei oder mehrere pharmazeutische Wirkstoffe enthalten.

Die Überzugsmittel können zur Beschichtung von Extrudaten, Minitabletten, Kapseln, Weichkapseln, Granulaten, Pellets, Mikropellets, Mikrokapseln, Nanokapseln oder Kristallen eingesetzt werden.

Zur Herstellung von Dosierungsformen können die überzogenen Granulate, Pellets, Mikropellets, Mikrokapseln, Kristalle mit geeigneten Hilfsstoffen abgemischt und zu Tabletten verpresst werden, die im wässrigen Milieu der Mundhöhle zerfallen und die gecoateten feinen Formlinge wieder freigeben. Besondere Bedeutung haben hierbei so genannte oral dispersibles, d. h. Tabletten, die im Mund innerhalb von kurzer Zeit zerfallen und die geschmacksmaskierten kleinen Formlinge freisetzen.

Weiterhin können die Überzugsmittel auch vorteilhaft zum Überziehen von Tabletten eingesetzt werden.

Wirkstoffklassen und Substanzen, die oftmals einen unangenehmen bitteren Geschmack hervorrufen können und sich vorteilhaft erfindungsgemäß formulieren lassen, sind z. B.:
Analgetika und Antirheumatika, wie Paracetamol, Diclofenac, Aceclofenac, Ibuprofen, Ketoprofen, Flubiprofen, Acetylsalicylsäure, Levacetylmethadol und Oxycodon;
Psychopharmaka, wie Promethazine, Donepezil, Modafinil, Nefazodon, Reboxetin, Sertindol und Sertralin;
Antibiotika, wie Erythromycin, Roxithromycin, Clarithromycin, Grepafloxacin, Ciprofloxacin, Levofloxacin, Sparfloxacin, Trovafloxacin und Nevirapin;
Betablocker, wie Propranolol, Metoprolol, Bisoprolol und Nebivolol;
Antidiabetika, wie Metformin, Miglitol und Repaglinid;
H₁-Antihistaminika, wie Diphenhydramin, Fexofenadin und Mizolastin;
H₂ Antihistaminika, wie Cimetidin, Famotidin, Roxatidin, Nizatidin, Ticlopidin, Cetirizin und Ranitidin;
Vitamine wie Thiaminnitrat sowie Chinidin-Sulfat, Amyloprilose-HCl, Pseudoephedrin-HCI, Sildenafil, Topiramat, Granisetron, Rebamipide, Chinin- HCl, etc.
Auch verschiedene Salze dieser Wirkstoffe können entsprechend formuliert werden.

Die hervorragende Geschmacksmaskierung resultiert aus der Unlöslichkeit der erfindungsgemäßen Polymeren bei pH-Werten größer 6 und der schnellen Löslichkeit bei pH-Werten unter 6. Das heißt im Speichel (pH-Wert: 7,2) sind entsprechend überzogene Formen sehr lange stabil und es erfolgt kein Kontakt des bitteren Arzneistoffs mit der Mundschleimhaut, aber im Magen bei pH-Werten von 1 bis 5 erfolgt eine sehr schnelle Freisetzung des Wirkstoffs. Die Auflösung ist dabei so schnell, dass kein Unterschied im Wirkungseintritt vorhanden ist, verglichen mit einer nicht gecoateten Form. In der Regel lösen sich Filmüberzüge eines erfindungsgemäßen Polymers innerhalb von 5 min in Magensaft auf, wohingegen sie in Phosphatpuffer pH 7,2 über 2 Stunden stabil sind. Überraschenderweise lösen sich die Filmüberzüge auch in Medien mit pH-Werten von 4,5 relativ schnell, so dass die daraus hergestellten Darreichungsformen auch bei anaciden Patienten bzw. Patienten, die mit Antacida behandelt werden, eine schnelle Wirkung entfalten. Diese hervorragenden Anwendungseigenschaften der Überzugsmittel bleiben auch nach der Überführung in Pulver und Redispergierung beziehungsweise Aufschmelzen der Pulver erhalten.

Überraschenderweise gelingt mit Hilfe des erfindungsgemäßen Verfahrens die Einarbeitung von in Wasser schwer löslichen Antioxidantien in die Polymerdispersionen, in der Art und Weise, dass die Antioxidantien in die Polymerteilchen wandern und nicht mehr partikulär in der Wasserphase vorliegen. Nur dadurch ist eine antioxidative Wirkung auf das Polymer und ein entsprechender Schutz zu erzielen.

Ferner gelingt überraschenderweise mit Hilfe des erfindungsgemäßen Verfahrens die Überführung der wässrigen Polymerdispersion in freifliessende Pulver, ohne das es zu größeren Agglomerationen und Ablagerungen in der Sprühvorrichtung kommt. Für den Fachmann war dies im Hinblick auf die Empfehlungen des Standes der Technik bezüglich Trocknungsmethode oder Temperaturführung der Trocknungsgase nicht zu erwarten gewesen. Überraschend war auch, dass der Einsatz hoher Scherkräfte von Vorteil ist, da der Fachmann bei hohen Scherkräften normalerweise ein Koagulieren der Dispersion erwartet hätte.

Das erfindungsgemäße Verfahren führt demgemäß zu Polymerpulvern mit einer guten Teilchengrößenverteilung und guten anwendungstechnischen Eigenschaften wie beispielsweise der Fliessfähigkeit. Bei der Verwendung zur Herstellung von Überzugsmitteln lassen sich die Pulver sehr vorteilhaft zu feinteiligen Dispersionen redispergieren.

### Beispiele

Verwendete Abkürzungen:
Glasübergangstemperatur: Tg

Alle Angaben in % beziehen sich auf Gew.-%.

Die Herstellung des Polymeren erfolgt analog Beispiel 1 der WO 2009/016258.
Polymer A: Methylmethacrylat/Diethylaminoethylmethacrylat, Gew.v. 60:40, K-Wert 50, Tg 62°C
Polymer B: Methylmethacrylat/Diethylaminoethylmethacrylat, Gew.v. 55:45, K-Wert 49, Tg 57°C
Polymer C: Methylmethacrylat/Diethylaminoethylmethacrylat, Gew.v. 53:47, K-Wert 52, Tg 55°C

Die K-Werte wurden 0.1 gew.-%ig in NMP gemessen .Die Polymere wurden als 30 gew.-%ige wässrige Dispersionen mit einem pH-Wert von 9+/- 0.3 eingesetzt. Die mittleren Partikelgrößen der Primärdispersionen betrugen 128, 127 und 131 nm. Die Glasübergangstemperaturen wurden mittels DSC bei einer Heizrate von 20 °K/min bestimmt. Die Mindestfilmbildetemperatur entsprach im Rahmen der Messgenauigkeit von plus/minus 5°C der Tg.

Bei der Bestimmung der mittleren Teilchengrößen der Pulver wurde der (d4,3-Wert) mittels Lichtbeugung mittels eines Malvern Mastersizers 2000 bestimmt.

Bei der Bestimmung der mittleren Teilchengrößen der redispergierten Pulver mittels Lichtstreuung wurde der Wert mittels eines "Malvern Zetasizer nano-s" als Intensitätsmittel bestimmt.

### Beispiel 1

3,0 g Butylhydroxytoluol wurden in 50 g Ethanol gelöst und unter Rühren in1000 ml einer wässrigen Dispersion des Polymers B mit einem Feststoffgehalt von 30% eingebracht. Anschließend wurden unter Rühren 72,9 ml 1 molarer Salzsäure eingemischt. Dies entspricht einem Neutralisationsgrad von 10 mol-%. Nach 0,5 Stunden Rühren wurde diese teilneutralisierte Dispersion in einem FSD-Sprühturm sprühgetrocknet, wobei die Zerstäubung über eine 1,2 mm Zweistoffdüse bei 2,5 bar Zerstäubungsdruck erfolgte. Die Zulufttemperatur betrug 115°C und die Ablufttemperatur 58°C. Der Feinanteil wurde während der Sprühtrocknung abgetrennt und wieder vor die Sprühdüse geblasen, so dass sprühgetrocknete Teilchen mit einer mittleren Teilchengröße von 170 µm resultierten.
Das sprühgetrocknete Produkt wurde in Wasser zu einer Sprühsuspension mit 20% Feststoffgehalt durch Rühren mit einem Blattrührer für 15 min redispergiert. Die Messung der Teilchengröße mittels Lichtstreuung ergab einen Wert von 135 nm.

### Beispiel 2

6,0 g Tocopherol wurden in 20,0g Cremophor RH 40 gelöst und anschließend wurde die Mischung mit 80,0g Wasser verdünnt. Dieses Solubilisat wurde langsam unter Rühren zu 1000 ml einer wässrigen Dispersion des Polymers A mit einem Feststoffgehalt von 30% gegeben, 1 Stunde weitergerührt und in einem-Sprühturm sprühgetrocknet. Die Zerstäubung erfolgte hierbei über eine 1,2 mm Zweistoffdüse bei 3,0 bar Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 107°C und die Ablufttemperatur 55°C. Die mittlere Teilchengröße der Pulver betrug 32 µm.
100g sprühgetrocknetes Produkt wurden in 900 ml Wasser eingetragen, in dem vorher 2,30g Bernsteinsäure gelöst wurden. Die Zubereitung wurde 20 min mit einem Propellerrührer gerührt. Die Messung der Teilchengröße mittels Lichtstreuung ergab einen Wert von 139 nm.

### Beispiel 3

4,5 g Butylhydroxytoluol wurden in 30,0 g Wasser gegeben, auf 80°C erwärmt, mit einem Ultra-turrax für 10 min bei 10.000 Upm feinst emulgiert und anschließend unter Rühren langsam zu 1000 ml einer 75°C heißen wässrigen Dispersion des Polymers A mit einem Feststoffgehalt von 30% gegeben. Diese Zubereitung wurde 1 Stunde weitergerührt und in einem-Sprühturm sprühgetrocknet. Die Zerstäubung erfolgte hierbei über eine 1,2 mm Zweistoffdüse bei 3,0 bar Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 108°C und die Ablufttemperatur 55°C. Die mittlere Teilchengröße der Pulver betrug 34 µm.

150g sprühgetrocknetes Produkt wurden in 850 ml Wasser eingetragen und die Zubereitung 20 min mit einem Ultra-turrax bei 12.000 Upm behandelt. Die Messung der Teilchengröße mittels Lichtstreuung ergab einen Wert von 230 nm.

### Beispiel 4

100 g Butylhydroxyanisol wurden in einem Extruder bei 140°C mit 400 g Soluplus zu einer festen Lösung verarbeitet. Die resultierenden Stränge wurden zu ca. 2 mm großen Stücken zerteilt. 20,0 g dieser festen Lösung wurden in 1000 ml einer wässrigen Dispersion des Polymers B mit einem Feststoffgehalt von 30% gegeben und es wurde für 1 Stunde weitergerührt. Danach wurden unter Rühren 36,5 ml 1 molarer Salzsäure zugesetzt. Dies entspricht einem Neutralisationsgrad von 5 mol-%. Diese teilneutralisierte Dispersion wurde in einem FSD-Sprühturm sprühgetrocknet, wobei die Zerstäubung über eine 1,2 mm Zweistoffdüse bei 3,0 bar Zerstäubungsdruck erfolgte. Die Zulufttemperatur betrug 125°C und die Ablufttemperatur 56°C. Der Feinanteil wurde während der Sprühtrocknung abgetrennt und wieder vor die Sprühdüse geblasen, so dass sprühgetrocknete Teilchen mit einer mittleren Teilchengröße von 180 µm resultierten. Während des Sprühprozesses wurde kolloidales Siliciumdioxid mit einer BET-Oberfläche von 200 m2/g in einer Menge von 0,5% bezogen auf die Gesamtmasse des Polymerpulvers in den Turm eingeblasen.
Das sprühgetrocknete Produkt wurde in Wasser zu einer Sprühsuspension mit 20% Feststoffgehalt durch Rühren mit einem Blattrührer für 20 min redispergiert. Die Messung der Teilchengröße mittels Lichtstreuung ergab einen Wert von 140 nm.

### Beispiel 5

5,0 g Butylhydroxytoluol und 10,0 g Dokusat-Natrium wurden zu 50,0 g Wasser gegeben und auf 50°C unter Rühren erwärmt. Nach Auflösen des Butylhydroxytoluols wurde diese Zubereitung mit 1000 ml einer wässrigen Dispersion des Polymers C mit einem Feststoffgehalt von 30% unter Rühren gemischt. Nach Zusatz von 1,31 g Malonsäure, was einem einem Neutralisationsgrad von 5 mol-% entspricht wurde diese teilneutralisierte Dispersion mit 200 ml einer 40%igen Suspension von Talkum vermischt und in einem FSD-Sprühturm sprühgetrocknet, wobei die Zerstäubung über eine 1,2 mm Zweistoffdüse bei 3,0 bar Zerstäubungsdruck erfolgte. Die Zulufttemperatur betrug 137°C und die Ablufttemperatur 59°C. Der Feinanteil wurde während der Sprühtrocknung abgetrennt und wieder vor die Sprühdüse geblasen, so dass sprühgetrocknete Teilchen mit einer mittleren Teilchengröße von 185 µm resultierten.
Das sprühgetrocknete Produkt wurde in Wasser zu einer Sprühsuspension mit 20% Feststoffgehalt durch Rühren mit einem Blattrührer für 15 min redispergiert. Die Messung der Teilchengröße mittels Lichtstreuung ergab für die Polymerteilchen einen Wert von 145 nm.

### Beispiel 6

100 g Polymerpulver hergestellt nach Beispiel 3 wurden mit 50 g feinst gemahlenem Talkum, 4g Indigotinlack und 2g Bernsteinsäure in einem Turbulamischer vermischt.
Nach der Redispergierung dieser Zubereitung in Wasser zu einer 15%igen Suspension mittels eines Propellerrührers ergab sich eine Teilchengröße der Polymerteilchen von 160 nm.

### Beispiel 7

Die nach Beispiel 6 hergestellte Zubereitung wurde mit 13,0 g Triethylcitrat versetzt, eine Stunde gerührt und durch Versprühen auf Tablettenkerne aufgebracht.

Sprühbedingungen:

| Maschine | Horizontaltrommelcoater |
|---|---|
| Zulufttemperatur | 54°C |
| Sprühdruck | 0.2 MPa |
| Formierluftdruck | 0.1 MPa |
| Sprühdüse | Schlick 930 / 1mm |
| Zuluftmenge | 200 m³/h |
| Sprührate | 30 g/min. |

Die resultierenden Filmtabletten wiesen einen glatten, glänzenden, geschmacksmaskierenden Überzug auf, der sich auch bei Stresslagerung unter 40°C nicht veränderte.

## Patentansprüche

1. Verfahren zur Herstellung von pulverförmigen, Antioxidantien enthaltenden Überzugsmitteln aus wässrigen Polymerdispersionen, enthaltend
als Komponente A ein durch radikalische Polymerisation erhaltenes Polymer aus
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,
**dadurch gekennzeichnet ist, dass** die Antioxidantien in Form einer Lösung oder einer Dispersion in die wässrige Polymerdispersion eingearbeitet werden und die wässrige Polymerdispersion durch Sprühverfahren in Pulverform überführt wird.

2. Verfahren nach Anspruch 1, **dadurch gekennzeichnet, dass** als Antioxidantien in Wasser schwerlösliche Antioxidantien, deren Löslichkeit in Wasser bei 20 °C nicht mehr als 1 g/l beträgt, eingesetzt werden.

3. Verfahren nach Anspruch 1 oder 2, **dadurch gekennzeichnet, dass** die Antioxidantien in Form einer Lösung in einem organischen Lösungsmittel oder als micellare, einen Solubilisator enthaltende wässrige Lösung eingebracht werden.

4. Verfahren nach einem der Ansprüche 1 bis 3, **dadurch gekennzeichnet, dass** die micellare Lösung der Antioxidantien als Solubilisatoren Tenside oder amphiphile Copolymere enthält.

5. Verfahren nach einem der Ansprüche 1 bis 4, **dadurch gekennzeichnet, dass** die Antioxidantien in Form einer festen Lösung der Antioxidantien in Tensiden oder Polymeren eingebracht werden.

6. Verfahren nach Anspruch 5, **dadurch gekennzeichnet, dass** die feste Lösung der Antioxidantien in Tensiden oder Polymeren durch Schmelzextrusion erhalten wird.

7. Verfahren nach einem der Ansprüche 1 bis 3, **dadurch gekennzeichnet, dass** die Antioxidantien in Form einer Emulgatoren mit einem Hydrophilic-Lipophilic-Balance-Wert größer 10 enthaltenden Dispersion in die wässrige Polymerdispersion eingearbeitet werden.

8. Verfahren nach einem der Ansprüche 1 bis 7, **dadurch gekennzeichnet, dass** die Antioxidantien in Mengen von 0,1 bis 10,0 Gew.-%, bezogen auf die Komponente A, eingearbeitet werden.

9. Verfahren nach einem der Ansprüche 1 bis 8, **dadurch gekennzeichnet, dass** die wässrige Polymerdispersion durch Sprühverfahren in Gegenwart eines Trocknungsgases in Pulver überführt wird, wobei die Eingangstemperatur des Trocknungsgases in die Sprühvorrichtung mindestens 20°C über der Glasübergangstemperatur, bestimmt nach der Methode wie beschrieben in der Beschreibung und mindestens 20°C über der Mindestfilmbildetemperatur, bestimmt nach der Methode wie beschrieben in der Beschreibung des Polymeren liegt und die Ausgangstemperatur des Trocknungsgases aus der Sprühvorrichtung bei 40 bis 85 °C gehalten wird.

10. Verfahren nach einem der Ansprüche 1 bis 9, **dadurch gekennzeichnet, dass** die Eingangstemperatur des Trocknungsgases in die Sprühvorrichtung mindestens 40°C über der Glasübergangstemperatur liegt und mindestens 40°C über der Mindestfilmbildetemperatur des Polymeren liegt.

11. Verfahren nach einem der Ansprüche 1 bis10, **dadurch gekennzeichnet, dass** die Ausgangstemperatur des Trocknungsgases aus der Sprühvorrichtung bei 45 bis 70°C gehalten wird.

12. Verfahren nach einem der Ansprüche 1 bis11, **dadurch gekennzeichnet, dass** die Eingangstemperatur des Trocknungsgases in die Sprühvorrichtung bei 100 bis 140°C und die Ausgangstemperatur des Trocknungsgases aus der Sprühvorrichtung bei 45 bis 70°C gehalten wird.

13. Verfahren nach einem der Ansprüche 1 bis12, **dadurch gekennzeichnet, dass** die Eingangstemperatur des Trocknungsgases in die Sprühvorrichtung bei 110 bis 130 °C und die Ausgangstemperatur des Trocknungsgases aus der Sprühvorrichtung bei 50 bis 60°C gehalten wird.

14. Verfahren nach einem der Ansprüche 1 bis13, **dadurch gekennzeichnet, dass** die Ausgangstemperatur des Trocknungsgases aus der Sprühvorrichtung im Bereich von plus/minus 5°C der Mindestfilmbildetemperatur gehalten wird.

15. Verfahren nach einem der Ansprüche 1 bis14, **dadurch gekennzeichnet, dass** das Sprühverfahren als Sprühtrocknung durchgeführt wird.

16. Verfahren nach einem der Ansprüche 1 bis14, **dadurch gekennzeichnet, dass** das Sprühverfahren als agglomerierende Sprühtrocknung durchgeführt wird.

17. Verfahren nach einem der Ansprüche 1 bis16, **dadurch gekennzeichnet, dass** der wässrigen Polymerdispersion vor dem Sprühverfahren eine Säure oder ein saures Salz zugesetzt wird.

18. Verfahren nach einem der Ansprüche 1 bis16, **dadurch gekennzeichnet, dass** nach dem Sprühverfahren dem entstandenen Pulver eine Säure oder ein saures Salz zugesetzt wird.

19. Verfahren nach einem der Ansprüche 1 bis18, **dadurch gekennzeichnet, dass** nach dem Sprühverfahren das entstandenen Pulver in Wasser redispergiert und mit einer Säure oder einem sauren Salz versetzt wird.

20. Verfahren nach einem der Ansprüche 1 bis19, **dadurch gekennzeichnet, dass** als Säure eine anorganische Säure oder ein saures Salz davon zugesetzt wird.

21. Verfahren nach einem der Ansprüche 1 bis20, **dadurch gekennzeichnet, dass** als Säure eine organische Säure oder ein saures Salz davon zugesetzt wird.

22. Verfahren nach einem der Ansprüche 1 bis21, **dadurch gekennzeichnet, dass** als Säure eine Säure oder ein saures Salz davon zugesetzt wird, die unter den Bedingungen des Sprühverfahrens zersetzt oder verdampft wird.

23. Verfahren nach einem der Ansprüche 1 bis22, **dadurch gekennzeichnet, dass** durch Zugabe der Säure oder des sauren Salzes der pH-Wert der wässrigen Dispersion, des Pulvers oder des in Wasser redispergierten Pulvers im Bereich von 5 bis 9 liegt.

24. Verfahren nach einem der Ansprüche 1 bis23, **dadurch gekennzeichnet, dass** durch Zugabe der Säure oder des sauren Salzes der pH-Wert der wässrigen Dispersion, des Pulvers oder des in Wasser redispergierten Pulvers im Bereich von 6 bis 8 liegt.

25. Verfahren nach einem der Ansprüche 1 bis24, **dadurch gekennzeichnet, dass** der wässrigen Polymerdispersion vor dem Sprühverfahren ein weiterer Hilfsstoff zugesetzt wird.

26. Verfahren nach einem der Ansprüche 1 bis25, **dadurch gekennzeichnet, dass** die Antioxidantien in Form einer Emulgatoren mit einem Hydrophilic-Lipophilic-Balance-Wert größer 10 enthaltenden Emulsion bei Temperaturen oberhalb des Schmelzpunktes des Antioxidans in die wässrige Polymerdispersion eingearbeitet werden.

## Claims

1. A process for producing pulverulent, antioxidant-comprising coating compositions of aqueous polymer dispersions, comprising
as component A, a polymer obtained by radical polymerization of
a) N,N-diethylaminoethyl methacrylate, and
b) at least one radically polymerizable compound selected from esters of α,β-ethylenically unsaturated mono- and dicarboxylic acids with C₁-C₈-alkanols,
wherein the antioxidants are incorporated into the aqueous polymer dispersion in the form of a solution or of a dispersion, and the aqueous polymer dispersion is converted to powder form by spraying processes.

2. The process according to claim 1, wherein sparingly water-soluble antioxidants whose solubility in water is not more than 1 g/l at 20°C are used as antioxidants.

3. The process according to claim 1 or 2, wherein the antioxidants are incorporated in the form of a solution in an organic solvent or as micellar aqueous solution comprising a solubilizer.

4. The process according to any one of claims 1 to 3, wherein the micellar solution of the antioxidants comprises surfactants or amphiphilic copolymers as solubilizers.

5. The process according to any one of claims 1 to 4, wherein the antioxidants are incorporated in the form of a solid solution of the antioxidants in surfactants or polymers.

6. The process according to claim 5, wherein the solid solution of the antioxidants in surfactants or polymers is obtained by melt extrusion.

7. The process according to any one of claims 1 to 3, wherein the antioxidants are incorporated into the aqueous polymer dispersion in the form of a dispersion comprising emulsifiers with a hydrophilic-lipophilic balance value greater than 10.

8. The process according to any one of claims 1 to 7, wherein the antioxidants are incorporated in amounts of from 0.1 to 10.0% by weight, based on component A.

9. The process according to any one of claims 1 to 8, wherein the aqueous polymer dispersion is converted to powder by spraying processes in the presence of a drying gas, where the entry temperature of the drying gas into the spraying apparatus is at least 20°C above the glass transition temperature, determined by the method as described in the description, and is at least 20°C above the minimum film-forming temperature, determined by the method as described in the description, of the polymer and the exit temperature of the drying gas from the spraying apparatus is kept at 40 to 85°C.

10. The process according to any one of claims 1 to 9, wherein the entry temperature of the drying gas into the spraying apparatus is at least 40°C above the glass transition temperature and is at least 40°C above the minimum film-forming temperature of the polymer.

11. The process according to any one of claims 1 to 10, wherein the exit temperature of the drying gas from the spraying apparatus is kept at 45 to 70°C.

12. The process according to any one of claims 1 to 11, wherein the entry temperature of the drying gas into the spraying apparatus is 100 to 140°C and the exit temperature of the drying gas from the spraying apparatus is kept at 45 to 70°C.

13. The process according to any one of claims 1 to 12, wherein the entry temperature of the drying gas into the spraying apparatus is kept at 110 to 130°C and the exit temperature of the drying gas from the spraying apparatus is kept at 50 to 60°C.

14. The process according to any one of claims 1 to 13, wherein the exit temperature of the drying gas from the spraying apparatus is kept in the range from plus/minus 5°C of the minimum film-forming temperature.

15. The process according to any one of claims 1 to 14, wherein the spraying process is carried out as spray drying.

16. The process according to any one of claims 1 to 14, wherein the spraying process is carried out as agglomerating spray drying.

17. The process according to any one of claims 1 to 16, wherein an acid or an acidic salt is added to the aqueous polymer dispersion before the spraying process.

18. The process according to any one of claims 1 to 16, wherein an acid or an acidic salt is added after the spraying process to the resulting powder.

19. The process according to any one of claims 1 to 18, wherein, after the spraying process, the resulting polymer is redispersed in water and admixed with an acid or an acidic salt.

20. The process according to any one of claims 1 to 19, wherein an inorganic acid or an acidic salt thereof is added as acid.

21. The process according to any one of claims 1 to 20, wherein an organic acid or an acidic salt thereof is added as acid.

22. The process according to any one of claims 1 to 21, wherein the acid added is an acid or an acidic salt thereof which is decomposed or evaporated under the conditions of the spraying process.

23. The process according to any one of claims 1 to 22, wherein, as a result of adding the acid or the acidic salt, the pH of the aqueous dispersion, of the powder or of the water-redispersed powder is in the range from 5 to 9.

24. The process according to any one of claims 1 to 23, wherein, by adding the acid or the acidic salt, the pH of the aqueous dispersion, of the powder or of the water-redispersed powder is in the range from 6 to 8.

25. The process according to any one of claims 1 to 24, wherein a further auxiliary is added to the aqueous polymer dispersion before the spraying process.

26. The process according to any one of claims 1 to 25, wherein the antioxidants are incorporated into the aqueous polymer dispersion in the form of an emulsion comprising emulsifiers with a hydrophilic-lipophilic balance value greater than 10 at temperatures above the melting point of the antioxidant.

## Revendications

1. Procédé de fabrication d'agents de revêtement en poudre contenant des antioxydants à partir de dispersions polymères aqueuses, contenant :
en tant que composant A, un polymère obtenu par polymérisation radicalaire de
a) du méthacrylate de N,N-diéthylaminoéthyle et
b) au moins un composé polymérisable par voie radicalaire, choisi parmi les esters d'acides mono- et dicarboxyliques α,β-éthyléniquement insaturés avec des alcanols en C₁-C₈,
**caractérisé en ce que** les antioxydants sont incorporés dans la dispersion polymère aqueuse sous la forme d'une solution ou d'une dispersion, et la dispersion polymère aqueuse est mise sous la forme de poudre par un procédé de pulvérisation.

2. Procédé selon la revendication 1, **caractérisé en ce que** des antioxydants difficilement solubles dans l'eau, dont la solubilité dans l'eau à 20 °C n'est pas supérieure à 1 g/l, sont utilisés en tant qu'antioxydants.

3. Procédé selon la revendication 1 ou 2, **caractérisé en ce que** les antioxydants sont introduits sous la forme d'une solution dans un solvant organique ou sous la forme d'une solution aqueuse micellaire contenant un solubilisateur.

4. Procédé selon l'une quelconque des revendications 1 à 3, **caractérisé en ce que** la solution micellaire des antioxydants contient des tensioactifs ou des copolymères amphiphiles en tant que solubilisateurs.

5. Procédé selon l'une quelconque des revendications 1 à 4, **caractérisé en ce que** les antioxydants sont introduits sous la forme d'une solution solide des antioxydants dans des tensioactifs ou des polymères.

6. Procédé selon la revendication 5, **caractérisé en ce que** la solution solide des antioxydants dans des tensioactifs ou des polymères est obtenue par extrusion par fusion.

7. Procédé selon l'une quelconque des revendications 1 à 3, **caractérisé en ce que** les antioxydants sont incorporés dans la dispersion polymère aqueuse sous la forme d'une dispersion contenant des émulsifiants ayant une valeur HLB (Hydrophilic-Lipophilic Balance) supérieure à 10.

8. Procédé selon l'une quelconque des revendications 1 à 7, **caractérisé en ce que** les antioxydants sont incorporés en quantités de 0,1 à 10,0 % en poids, par rapport au composant A.

9. Procédé selon l'une quelconque des revendications 1 à 8, **caractérisé en ce que** la dispersion polymère aqueuse est mise sous la forme de poudre par un procédé de pulvérisation en présence d'un gaz siccatif, la température d'entrée du gaz siccatif dans le dispositif de pulvérisation étant au moins 20 °C au-dessus de la température de transition vitreuse, déterminée par la méthode telle que décrite dans la description, et au moins 20 °C au-dessus de la température filmogène minimale, déterminée par la méthode telle que décrite dans la description, du polymère, et la température de sortie du gaz siccatif du dispositif de pulvérisation étant maintenue de 40 à 85 °C.

10. Procédé selon l'une quelconque des revendications 1 à 9, **caractérisé en ce que** la température d'entrée du gaz siccatif dans le dispositif de pulvérisation est au moins 40 °C au-dessus de la température de transition vitreuse et au moins 40 °C au-dessus de la température filmogène minimale du polymère.

11. Procédé selon l'une quelconque des revendications 1 à 10, **caractérisé en ce que** la température de sortie du gaz siccatif du dispositif de pulvérisation est maintenue de 45 à 70 °C.

12. Procédé selon l'une quelconque des revendications 1 à 11, **caractérisé en ce que** la température d'entrée du gaz siccatif dans le dispositif de pulvérisation est maintenue de 100 à 140 °C et la température de sortie du gaz siccatif du dispositif de pulvérisation de 45 à 70 °C.

13. Procédé selon l'une quelconque des revendications 1 à 12, **caractérisé en ce que** la température d'entrée du gaz siccatif dans le dispositif de pulvérisation est maintenue de 110 à 130 °C et la température de sortie du gaz siccatif du dispositif de pulvérisation de 50 à 60 °C.

14. Procédé selon l'une quelconque des revendications 1 à 13, **caractérisé en ce que** la température de sortie du gaz siccatif du dispositif de pulvérisation est maintenue dans la plage de plus/moins 5 °C de la température filmogène minimale.

15. Procédé selon l'une quelconque des revendications 1 à 14, **caractérisé en ce que** le procédé de pulvérisation est réalisé sous la forme d'un séchage par pulvérisation.

16. Procédé selon l'une quelconque des revendications 1 à 14, **caractérisé en ce que** le procédé de pulvérisation est réalisé sous la forme d'un séchage par pulvérisation agglomérant.

17. Procédé selon l'une quelconque des revendications 1 à 16, **caractérisé en ce qu'**un acide ou un sel acide est ajouté à la dispersion polymère aqueuse avant le procédé de pulvérisation.

18. Procédé selon l'une quelconque des revendications 1 à 16, **caractérisé en ce qu'**un acide ou un sel acide est ajouté à la poudre formée après le procédé de pulvérisation.

19. Procédé selon l'une quelconque des revendications 1 à 18, **caractérisé en ce que** la poudre formée est redispersée dans de l'eau après le procédé de pulvérisation et mélangée avec un acide ou un sel acide.

20. Procédé selon l'une quelconque des revendications 1 à 19, **caractérisé en ce qu'**un acide inorganique ou un sel acide de celui-ci est ajouté en tant qu'acide.

21. Procédé selon l'une quelconque des revendications 1 à 20, **caractérisé en ce qu'**un acide organique ou un sel acide de celui-ci est ajouté en tant qu'acide.

22. Procédé selon l'une quelconque des revendications 1 à 21, **caractérisé en ce qu'**un acide ou un sel acide de celui-ci qui se décompose ou s'évapore dans les conditions du procédé de pulvérisation est ajouté en tant qu'acide.

23. Procédé selon l'une quelconque des revendications 1 à 22, **caractérisé en ce que** le pH de la dispersion aqueuse, de la poudre ou de la poudre redispersée dans de l'eau se situe dans la plage allant de 5 à 9 par ajout de l'acide ou du sel acide.

24. Procédé selon l'une quelconque des revendications 1 à 23, **caractérisé en ce que** le pH de la dispersion aqueuse, de la poudre ou de la poudre redispersée dans de l'eau se situe dans la plage allant de 6 à 8 par ajout de l'acide ou du sel acide.

25. Procédé selon l'une quelconque des revendications 1 à 24, **caractérisé en ce qu'**un adjuvant supplémentaire est ajouté à la dispersion polymère aqueuse avant le procédé de pulvérisation.

26. Procédé selon l'une quelconque des revendications 1 à 25, **caractérisé en ce que** les antioxydants sont incorporés dans la dispersion polymère aqueuse sous la forme d'une émulsion contenant des émulsifiants ayant une valeur HLB (Hydrophilic-Lipophilic Balance) supérieure à 10 à des températures supérieures au point de fusion de l'antioxydant.
